# EUROPÄISCHE PATENTSCHRIFT

(11) **EP 0 409 149 B1**
(45) Veröffentlichungstag und Bekanntmachung des Hinweises auf die Patenterteilung: **27.04.1994**
(21) Anmeldenummer: 90113619.2
(22) Anmeldetag: 16.07.1990
(51) Int. Cl.: A61F 13/15

(54) **Saugfähiger Wegwerfartikel**
Disposable absorbent article
Article absorbant à jeter

(30) Priorität: 17.07.1989 JP 84183/89
(43) Veröffentlichungstag der Anmeldung: 23.01.1991
(73) Patentinhaber: UNI-CHARM CORPORATION, Kawanoe-shi Ehime-ken (JP)
(72) Erfinder: Suzuki, Migaku, Kanagawa-ken (JP); Nozaki, Satoshi, Uma-gun, Ehime-ken (JP); Kudo, Takeshi, Kawanoe-shi, Ehime-ken (JP); Ohnishi, Kazuaki, Kakegawa-shi, Shizuoka-ken (JP); Imai, Shigeo, Iyomishima-shi, Ehime-ken (JP)
(74) Vertreter: Sperling, Rüdiger, Dipl.-Ing.

(56) Entgegenhaltungen:
- EP-A- 0 243 013
- GB-A- 2 181 336
- GB-A- 2 193 625

## Beschreibung

### Hintergrund der Erfindung.

Die Erfindung betrifft einen saugfähigen Wegwerfartikel, insbesondere solche Artikel wie beispielsweise Wegwerfwindeln, mit einer flüssigkeitsdurchlässigen oberen Lage, einer flüssigkeitsundurchlässigen rückwärtigen Lage, einem Saugkern, der zwischen der oberen und der rückwärtigen Lage sandwichartig eingelegt ist, sich jeweils von den Seitenrändern des Saugkerns nach außen erstreckenden ersten Klappen und mit zweiten Klappen, die den ersten Klappen zugeordnet sind und einerseits jeweils einen Abschnitt aufweisen, der von der oberen Flache der zugeordneten ersten Klappe eine Abzweislinie bildend nach oben ragt und teilweise elastisch ist, um als Flüssigkeitssperre zu wirken, und andererseits einen Abschnitt hat, der sich von der Abzweiglinie horizontal nach außen erstreckt, wobei der Abschnitt jeder zweiten Klappe, der sich horizontal von der Abzweiglinie erstreckt, auf der Oberfläche mit einem flüssigkeitsabsorbierenden Mittel versehen ist.

In den japanischen Patentanmeldungen Veröffentlichungs-Gazette Nr. 1984-25741 sowie 1987-25020 wurde bereits vorgeschlagen, saugfähige Artikel nicht nur mit elastischen Klappen zu versehen, die sich von einander gegenüberliegenden Rändern nach außen erstrecken, sondern auch mit zusätzlichen Klappen, die von der oberen Fläche des saugfähigen Gegenstands an Stellen innerhalb der sich gegenüberliegenden Seitenränder nach oben erstrecken, wobei diese zusätzlichen Klappen als Flüssigkeitssperren dienen und teilweise elastisch sind, so daß diese zusätzlichen Klappen im wesentlichen jede beliebige Menge von Urin am Ausfließen aus dem saugfähigen Gegenstand hindern und diese Flüssigkeitsmenge in den Klappen halten. Eine solche Anordnung ist sicherlich wirkungsvoll zum Verhindern von Auslaufen der Körperflüssigkeit, jedoch nachteilig wie sich auch den folgenden Abhandlungen der Dokumente GB-A-21 81 336 u. EP-A-02 43 013 entnehmen läßt.

Aus der GB-A 21 81 336 ist ein saugfähiger Wegwerfartikel, nämlich eine Wegwerfwindel bekannt, die einen Saugkern hat, der zwischen einer oberen und einer unteren Lage sandwichartig eingelegt ist, wobei die obere Lage flüssigkeitsdurchlässig und die untere Lage flüssigkeitsundurchlässig ist.

Die obere und die untere Lage dieser vorbekannten Wegwerfwindel sind an den Schmalseiten des Saugkerns durch einen Kleber miteinander verbunden, so daß sie eine erste Klappe bilden. Unmittelbar im Adhäsionspunkt der beiden Lagen ist eine zweite Klappe angeordnet, die als Flüssigkeitssperre wirken soll, so daß Körperflüssigkeit, die nicht von dem Saugkern aufgenommen wird, nicht aus der Windel austritt.

Bei dieser vorbekannten Wegwerfwindel erstreckt sich die erste Klappe nach außen und ist die zweite Klappe der ersten Klappe zugeordnet, wobei diese zweite Klappe nach oben ragt und teilweise elastisch ist.

In der Figur 7 dieser Druckschrift ist eine zweite Ausführungsform dieser Wegwerfwindel dargestellt, bei der die zweite nach oben ragende Klappe aus der oberen Lage gebildet ist. Da diese obere Lage jedoch flüssigkeitsdurchlässig sein muß, bietet diese obere Klappe keinen ausreichenden Schutz gegen auslaufende Körperflüssigkeit. Diesen Nachteil weist auch die Ausführungsform gemäß Figur 6 dieser Druckschrift auf, obwohl in dieser Ausführungsform die flüssigkeitsundurchlässige rückwärtige Lage in die zweite Klappe integriert ist. Da diese Lage jedoch nur einen Abschnitt der zweiten Klappe abdeckt, besteht auch hier die Gefahr von austretender Körperflüssigkeit.

In der EP-A 02 43 013 ist eine Wegwerfwindel offenbart, welche aus einem Saugkern besteht, der zwischen einer oberen und einer rückwärtigen Lage sandwichartig eingelegt ist, wobei die obere Lage flüssigkeitsdurchlässig und die rückwärtige Lage flüssigkeitsundurchlässig ausgebildet sind. An den Seitenrändern des Saugkerns ist jeweils eine sich nach außen erstreckende erste Klappe angeordnet, die durch Zusammenkleben der oberen Lage mit der rückwärtigen Lage hergestellt ist. Auf dieser ersten Klappe ist eine zweite Klappe angeordnet, die im Querschnitt im wesentlichen l-förmig ausgebildet ist, so daß ein Schenkel dieser Klappe im wesentlichen nach oben ragt. Diese zweite Klappe dient als Flüssigkeitssperre und ist teilweise elastisch, so daß diese Klappe im wesentlichen jede beliebige Menge von Urin am Ausfließen aus der Windel hindert und diese Flüssigkeitsmenge zwischen den Klappen hält.

Nachteil dieser vorbekannten Windel ist jedoch, daß die Flüssigkeitsmenge, die in dem Raum zwischen den zusätzlichen Klappen gehalten ist und die sich an den nach außen erstreckenden elastischen Klappen sammelt, Hautreizungen und Ekzeme sowie Aufscheuern verursacht, da keine flüssigkeitsaufsaugenden Komponenten in dem Raum vorhanden sind und die Haut des Trägers in Kontakt mit der darin gehaltenen Urinmenge verbleibt.

Der Erfindung liegt die Aufgabe zugrunde, eine Wegwerfwindel der genannten Gattung anzugeben, die bei einfachem und kostengünstigem Aufbau auch in gefülltem Zustand über eine längere Zeit hinweg die Haut des Trägers frei von Hautreizungen hält.

Dadurch, daß die Klappen, die nach oben abzweigen und teilweise elastisch sind, um als Flüssigkeitssperre zu dienen, an der oberen Fläche der sich von den jeweiligen Abzweiglinien horizontal nach außen erstreckenden Abschnitten mit flüssigkeitsabsorbierendem Mittel versehen sind, werden die oben erwähnten Nachteile vermieden, da die Flüssigkeit an den kritischen Stellen absorbiert wird.

Die Erfindung im Überblick.

Bei einem saugfähigen Wegwerfartikel, wie beispielsweise Wegwerfwindeln, mit einer flüssigkeitsdurchlässigen oberen Lage, einer flüssigkeitsundurchlässigen rückwärtigen Lage, einem Saugkern, der zwischen der oberen und der rückwärtigen Lage sandwichartig eingelegt ist, sich jeweils von den Seitenrändern des Saugkerns nach außen erstreckenden ersten Klappen und zweiten Klappen, die den ersten Klappen zugeordnet sind und jeweils einen Abschnitt aufweisen, der von der oberen Fläche der zugeordneten ersten Klappe nach oben ragt und teilweise elastisch ist, um als Flüssigkeitssperre zu wirken, einerseits, und andererseits einem Abschnitt, der sich von der Abzweiglinie nach außen erstreckt, wird die Aufgabe dadurch gelöst, daß jede zweite Klappe, die sich horizontal von der Abzweiglinie nach außen erstreckt, auf der Oberfläche mit einem flüssigkeitsabsorbierenden Mittel versehen ist.

Kurze Beschreibung der Figuren.

Es zeigen:
Fig. 1 eine Draufsicht auf eine abgewickelte Windel als ein Beispiel für den erfindungsgemäß aufgebauten Artikel,
Fig. 2 eine Teilansicht eines Schnitts entlang der Linie II-II in Fig. 1,
Fig. 3 eine Teilansicht ähnlich derjenigen aus Fig. 2, mit einer Darstellung eines weiteren Ausführungsbeispiels,
Fig. 4 eine Teilansicht ähnlich derjenigen aus Fig. 2, mit einer Darstellung eines nächsten Ausführungsbeispiels und
Fig. 5 eine Teilansicht ähnlich derjenigen aus Fig. 2, mit einer Darstellung eines weiteren Ausführungsbeispiels.

Bevorzugtes Ausführungsbeispiel der Erfindung.

Die vorliegende Erfindung wird nachfolgend beispielhaft für die Ausführungsform einer Wegwerfwindel anhand der begleitenden Zeichnungen näher erläutert.

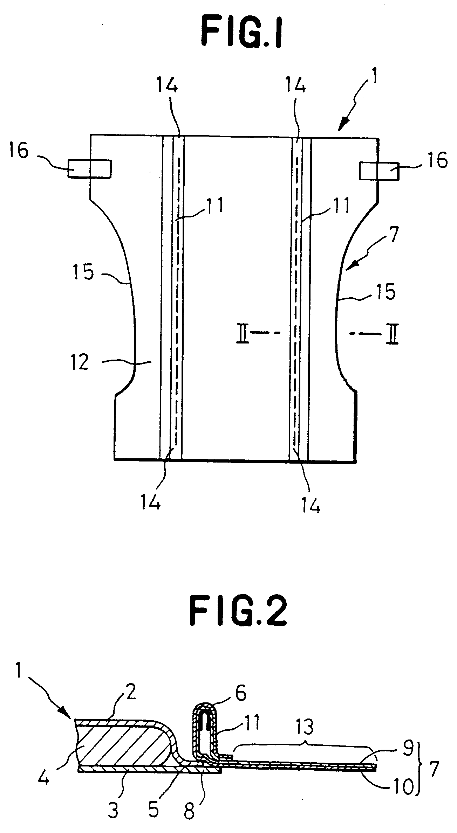

Es wird bezug genommen auf die Fig. 1 und 2. Die Windel 1 weist eine flüssigkeitsdurchlässige obere Lage 2 auf, die in Kontakt mit der Haut des Trägers kommt, wenn die Windel angelegt ist, eine flüssigkeitsundurchlässige rückwärtige Lage 3, die nicht in Kontakt mit der Haut des Trägers kommt, einen flüssigkeitsabsorbierender Kern 4, der zwischen diesen beiden Lagen 2 und 3 sandwichartig eingelegt ist, erste Klappen 5, die sich von den jeweiligen Seitenrändern des Saugkerns nach außen erstrecken und zweite Klappen 7, die mit den äußeren Rändern 8 der jeweiligen ersten Klappe verbunden sind und elastische Teile 6 aufweisen.

Jede der zweiten Klappen 7 ist eine kombinierte Lage aus einer flüssigkeitsabsorbierenden Lagenschicht 9 und einer flüssigkeitsundurchlässigen, aber feuchtigkeitsdurchlässigen Lagenschicht 10, so daß eine solche kombinierte Lage vollständig als eine Flüssigkeitssperre dient. Ein Ende der zweiten Klappe 7 wird zurückgefaltet, um eine Hülle 11 zu bilden, und der Rest der Klappe 7, d.h. ein Abschnitt, der durch die Hülle 11 definiert wird, erstreckt sich von der Hülle 11 nach außen, wobei ein Abschnitt 13 davon ein flüssigkeitsabsorbierendes Mittel auf seiner Oberfläche besitzt.

Ein im Schnitt in Form eines umgedrehten U ausgeformten elastisches Teil ist in der Hülle 11 angeordnet, um in Längsrichtung der Hülle elastisch zu sein und um dabei die Hülle 11 mit einer Elastizität in Längsrichtung auszustatten. Insbesondere ist die Hülle 11 unter seiner zusammenziehenden Kraft in aufrechter Lage gehalten. Die in Längsrichtung einander gegenüberliegenden Enden 14 der Hülle 11 sind mehrfach nach innen zusammengelegt und auf die zugeordnete erste Klappe geklebt. Es ist auch möglich, daß die genannten einander gegenübeliegenden Enden nach außen umgelegt werden und auf den äußeren Bereich 13 der zweiten Klappe geklebt werden oder daß eines der Enden 14 nach innen und auf die erste Klappe geklebt, während das andere Ende 14 nach außen umgelegt und auf den äußeren Abschnitt 13 der zweiten Klappe geklebt wird.

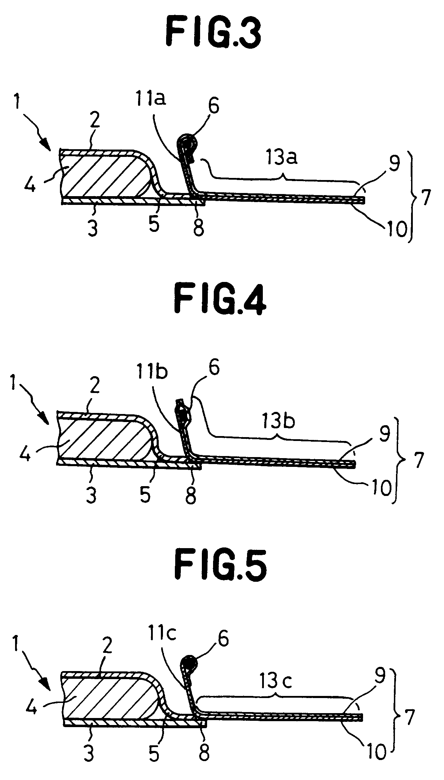

Bei dem in Fig. 3 dargestellten Ausführungsbeispiel weist die zweite Klappe 7 ein um das elastische Teil 6 herumgerolltes Ende auf, wobei der nach oben gerichtete Abschnitt 11a und der Abschnitt 13a an ihren jeweiligen Oberflächen mit dem flüssigkeitsabsorbierenden Mittel versehen sind.

Bei dem in Fig. 4 dargestellten Ausführungsbeispiel weist die zweite Klappe 7 ein Ende auf, das das elastische Teil 6 sandwichartig zwischen den beiden Lagen 9, 10 einschließt, wobei der nach oben gerichtete Abschnitt 11b und der Abschnitt 13b jeweils an seiner Oberfläche mit dem flüssigkeitsabsorbierenden Mittel ausgerüstet ist.

Bei dem in Fig. 5 dargestellten Ausführungsbeispiel besitzt die zweite Klappe 7 ein Ende, von dem ein unmittelbar an diesem Ende gelegener Teil lediglich aus der Lagenschicht 10 besteht, wobei dieses Ende um das elastische Teil herumgerollt ist und der nach oben abzweigende Abschnitt 11c und der Abschnitt 13c auf der jeweiligen Oberfläche mit dem flüssigkeitsabsorbierenden Mittel versehen ist. Mit Ausnahme der Ausbildung der jeweiligen zweiten Klappen sind die in den Figuren 3 bis 5 dargestellten Ausführungsformen identisch mit dem in Fig. 2 dargestellten Ausführungsbeispiel. Bei den in den Figuren 2 bis 5 dargestellten Ausführungsformen sind die zweiten Klappen 7 jeweils entlang der Außenränder mit gekrümmten Linien 15 geformt, wobei die in Längsrichtung einander gegenüberliegenden Enden, wie in Fig. 1 dargestellt, intakt bleiben. Des weiteren sind die zweiten Klappen 7 jeweils neben ihren oberen Enden, wie in Fig. 1 dargestellt, entsprechend den einander gegenüberliegenden Seiten der rückwärtigen Taile jeweils mit Befestigungsklebestreifen ausgestattet.

Obwohl nicht dargestellt, können auch entlang der gekrümmten Linie 15 der jeweiligen zweiten Klappen 7 elastische Teile vorgesehen sein und die nach oben abzweigenden Abschnitte 11, 11a, 11b und 11c der jeweiligen zweiten Klappen 7 können auch in V-Form ausgebildet sein. Die obere Lage 2 kann aus flüssigkeitsdurchlässigem Faservlies, porösem Kunststoffilm oder ähnlichem hergestellt sein, die rückwärtige Lage 3 demgegenüber aus flüssigkeitsundurchlässigem Kunststoffilm, flüssigkeitsundurchlässigem und feuchtigkeitsdurchlässigem Kunststoffilm oder ähnlichem und der Kern 4 kann aus einer flockigen Pulpe, gemischt mit supersaugfähigen Polymerpartikeln oder ähnlichem bestehen. Die Lage 9 kann sein ein schweißabsorbierendes Faservlies, wie beispielsweise spinnverklebtes (spun-bonded) Vlies, unter Wärmebehandlung schmelzverklebtes Vlies oder blasgeschmolzenes Vlies, das hergestellt wird durch Extrusion mittels Luftstrahl, bestehend aus Polyesterfasern mit hydrophilischer Oberflächenbehandlung; es können hydrophilische Fasern vom Cellulosetyp, wie Rayon, sein, wobei diese Fasern eine hydrophilische Oberflächenbehandlung erfahren haben und unter Einwirkung von unter hohem Druck stehenden Fluid, wie beispielsweise einem Wasserstrahl, mit einem Faservlies aus hydrophobischen Fasern, wie beispielsweise Polyester, Polypropylen oder ähnlichem verschlungen worden sind und es kann sich handeln um ein schmelzverbundenes Faservlies, bestehend aus einer Mischung von Fasern, denen die genannte hydrophilische Oberflächenbehandlung zuteil geworden ist oder hydrophilischen Fasern mit den hydrophobischen Fasern. Die Fasern, die eine hydrophilische Oberflächenbehandlung erfahren haben, oder die hydrophilischen Fasern, die mit den hydrophobischen Fasern gemischt werden sollen, nehmen vorzugsweise 70% oder mehr des Faservliesendprodukts ein. Die Lagenschicht 10 kann aus einem Faservlies hergestellt sein, wie beispielsweise spinnverklebtes (spun-bonded) Faservlies, unter Wärmebehandlung hergestellte schmelzgeblasene Vlies oder schmelzgeblasenes Vlies, hergestellt durch Extrusion unter Zuhilfenahme von Luftstrahlen mit einer Flüssigkeitssperrencharakteristik und Feuchtigkeitsdurchlässigkeit, wie beispielsweise Polyester oder Polypropylen oder mikroporöser Kunststoffilm aus Polyethylen oder ähnlichem.

## Patentansprüche

1. Saugfähiger Wegwerfartikel, insbesondere solche Artikel wie beispielsweise Wegwerfwindeln (1), mit einer flüssigkeitsdurchlässigen oberen Lage (2), einer flüssigkeitsundurchlässigen rückwärtigen Lage (3), einem Saugkern (4), der zwischen der oberen und der rückwärtigen Lage (2,3) sandwichartig eingelegt ist, sich jeweils von den Seitenrändern des Saugkerns (4) nach außen erstreckenden ersten Klappen (5) und mit zweiten Klappen (7), die den ersten Klappen (5) zugeordnet sind und einerseits jeweils einen Abschnitt (11) aufweisen, der von der oberen Fläche (10) der zugeordneten ersten Klappe (5) eine Abzweiglinie bildend nach oben ragt und teilweise elastisch ist, um als Flüssigkeitssperre zu wirken, und andererseits einen Abschnitt (13) hat, der sich von der Abzweiglinie horizontal nach außen erstreckt, wobei der Abschnitt (13) jeder zweiten Klappe (7), der sich horizontal von der Abzweiglinie erstreckt, auf der Oberfläche mit einem flüssigkeitsabsorbierenden Mittel versehen ist,
**dadurch gekennzeichnet**,
daß zumindest der Abschnitt (13) jeder zweiten Klappe (7) eine kombinierte Lage ist, bestehend aus einer flüssigkeitsabsorbierenden Lagenschicht (9), welche die Oberseite bildet, und einer flüssigkeitsundurchlässigen, aber feuchtigkeitsdurchlässigen Lagenschicht (10), welche die Unterseite bildet und
daß die flüssigkeitsundurchlässige, jedoch feuchtigkeitsdurchlässige Lagenschicht (10) aus einem Faservlies oder einem Kunststoffilm mit Mikroporen besteht.

2. Wegwerfartikel nach Anspruch 1, dadurch gekennzeichnet, daß die flüssigkeitsabsorbierende Lagenschicht (9) aus Faservlies besteht.

3. Wegwerfartikel nach Anspruch 1 oder nach Anspruch 2, dadurch gekennzeichnet, daß die zweiten Klappen (7) zumindest in ihrem oberen Ende mit einem elastischen Teil (6) versehen sind, so daß jedes der elastischen Teile (6) durch einen Abschnitt der zugeordneten zweiten Klappe (7) bedeckt ist.

## Claims

1. Disposable absorbent article, in particular an article such as, for example, a disposable nappy (1), comprising a liquid-permeable upper layer (2), a liquid-impermeable rear layer (3), an absorbent core (4) inserted sandwich-like between the upper and rear layers (2,3), first flaps (5) extending outwards from the side edges of the absorbent core (4), and second flaps (7) which are disposed towards the first flaps (5) and on one side each have a portion (11) which forms a branch from the corresponding first portion (7), extends upwards and is partially elastic, in order to act as a liquid barrier, and on the other side has a portion (13) which extends horizontally outwards from the branch, the portion (13) of each second flap (7), which extends horizontally from the branch, being provided on its surface with a liquid-absorbing material,
characterised in that at least the portion (13) of each second flap (7) is a combination of layers, comprising a liquid-absorbing layer (9) forming the upper side, and a liquid-impermeable, but moisture-permeable, layer (10) forming the lower side, and in that the liquid-impermeable, but moisture-permeable, layer (10) comprises a fibre fleece or a plastics film with micropores.

2. Disposable article as claimed in Claim 1, characterised in that the liquid-absorbing layer (9) comprises fibre fleece.

3. Disposable article as claimed in Claim 1 or Claim 2, characterised in that the second flaps (7) are provided at least at their upper ends with an elastic part (6), so that each of the elastic parts (6) is covered by a portion of the corresponding second flap (7).

## Revendications

1. Article absorbant à jeter, en particulier un article tel que par exemple les couches à jeter (1), avec un pli supérieur imperméable au liquide, (2) un pli arrière imperméable au liquide (3), un noyau absorbant (4), qui est intercalé en sandwich entre les plis supérieur et arrières (2,3), des premiers rabats (5) s'étendant depuis chacun des côtés latéraux du noyau absorbant (4) vers l'extérieur et avec des deuxièmes rabats (7) qui sont associés aux premiers rabats (5) et présentent chacun d'une part une découpe (11), laquelle se dirige vers le haut depuis la face supérieure (10) du premier rabat associé (5) en formant une ligne de dérivation et est partiellement élastique pour faire office de barrière contre les fuites, et possède d'autre part une découpe (13) laquelle s'étend horizontalement vers l'extérieur depuis la ligne de dérivation, la découpe (13) de chacun des deuxièmes rabats (7), qui s'étend horizontalement depuis la ligne de dérivation, étant dotée sur la face supérieure d'un moyen absorbant le liquide,
caractérisé en ce que
la découpe (13) au moins de chacun des deuxièmes rabats (7) constitue un pli combiné , comprenant une couche absorbant le liquide (9), laquelle forme la face supérieure, et une couche imperméable au liquide mais perméable à l'humidité (10), qui constitue la face inférieure, et en ce que la couche imperméable au liquide mais perméable à l'humidité (10) est composée d'un tissu ou d'un film synthétique à micropores.

2. Article à jeter selon la revendication 1, caractérisé en ce que la couche absorbant l'humidité (9) est composée de tissu.

3. Article à jeter selon la revendication 1 ou la revendication 2, caractérisé en ce que les deuxièmes rabats (7) sont dotés à leur extrémité supérieure au moins d'un élément élastique (6), de sorte que chacun des éléments élastiques (6) soit recouvert par une découpe des deuxièmes rabats associés (7).
